(19) Europäisches Patentamt
European Patent Office
Office européen des brevets

(11) Publication number: 0 021 445
B1

(12) # EUROPEAN PATENT SPECIFICATION

(45) Date of publication of patent specification: 18.01.84

(21) Application number: 80103669.0

(22) Date of filing: 27.06.80

(51) Int. Cl.$^3$: C 01 B 33/28, B 01 J 29/28, C 10 G 11/05, C 10 G 47/16, C 10 G 35/095, C 07 C 5/22, C 07 C 5/41, C 07 C 2/12, C 07 C 2/54, C 07 C 6/00, C 07 C 4/10

(54) **Method for preparing zeolites, zeolites obtained by such method and their use for the conversion of hydrocarbons.**

(30) Priority: 29.06.79 IT 2397879

(43) Date of publication of application:
07.01.81 Bulletin 81/1

(45) Publication of the grant of the patent:
18.01.84 Bulletin 84/3

(84) Designated Contracting States:
BE DE FR GB IT NL

(56) References cited:
EP - A - 0 000 432
US - A - 3 702 886

HELVETICA CHEMICA ACTA, vol. 57, Fascicule 6, 1974, Basel, CH W. SIEBER et al. "Formation and properties of Losod, a new sodium zeolite", pages 1533-49

(73) Proprietor: Montedison S.p.A.
Patents & Licensing Dept. Foro Buonaparte, 31
P.O. Box 10528
I-20121 Milan (IT)

(72) Inventor: Le Van Mao, Raymond
77, Via Cenisio
Milan (IT)
Inventor: Pilati, Orlando
14, Via Valparaiso
Milan (IT)
Inventor: Moretti, Enrico
24, Via Matteotti
Bollate, Milan (IT)
Inventor: Covini, Romano
6, Via S. Simpliciano
Milan (IT)
Inventor: Genoni, Fausto
20, Via Giuseppe Verdi
Samarate, Varese (IT)

(74) Representative: Schmied-Kowarzik, Volker, Dr. et al,
Patentanwälte Dr. V. Schmied-Kowarzik Dipl.-Ing. G. Dannenberg Dr. P. Weinhold Dr. D. Gudel Dipl.-Ing. S. Schubert Siegfriedstrasse 8
D-8000 München 40 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 021 445 B1

# Method for preparing zeolites, zeolites obtained by such method and their use for the conversion of hydrocarbons

The invention relates to a method for preparing zeolites showing considerable catalytic properties towards hydrocarbon conversions.

The prior art describes many synthetic zeolites, different methods of preparing same and the use of synthetic or natural zeolites in the catalytic conversion of hydrocarbons; the zeolites obtained by our new method are characterized by a high stability to heat and acids, what renders them particularly suited for processes involving high temperatures, such as the isomerization of xylenes; other advantages will appear from the following description.

In its most general embodiment, the invention relates to a method for preparing zeolites having the formula:

$$(1.5 \pm 0.6)\ M_{2/n}\ O.Al_2O_3.YSiO_2.Z\ H_2O$$

where Y ranges from 20 to 90, preferably from 50 to 80, Z ranges from 2 to 12, preferably from 3 to 8, M is at least a cation and n is the valence of cation M, and where aluminium and silicon may be optionally substituted, at least partially, by gallium and germanium respectively. The original cations can be replaced, at least partially, by other cations, employing well known ion exchange techniques. Cation M is preferably selected from the monovalent cations, the cations of the elements of rare earths, the cations of the elements of 2nd and 8th Groups of the Periodic System and mixtures thereof; in particular the cations can be advantageously selected from the alkaline ions, the hydrogen ion, the ammonium ion, the diethyl-piperidinium ion and mixtures thereof.

The formula of diethyl-piperidinium ion $(DEPP)^+$ is:

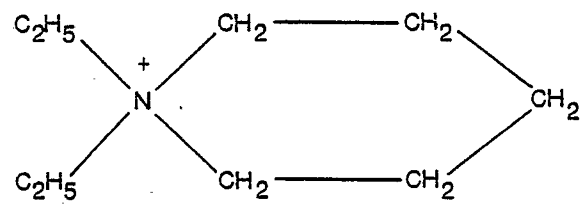

In various cases, in particular when zeolites must be employed as a catalyst, it is advisable to mix the zeolite with a binder such as silica, alumina, clays (in particular bentonite), various metal oxides and in general all the binders considered in US patent 3,702,886.

The method according to the invention comprises the admixture of diethyl-piperidinium hydroxide or a salt thereof with water and with:

a) at least one compound containing sodium;

b) at least one compound containing a metal selected from the group comprising aluminium, gallium and mixtures thereof;

c) at least one compound containing an element selected from the group comprising silicon, germanium and mixtures thereof;

the respective molar ratios, expressed as oxides ratios, being comprised in the following ranges:

$SiO_2 : Al_2O_3$ = from 20 to 120 (preferably from 50 to 85)

$Na_2O : SiO_2$ = from 0.07 to 0.50 (preferably from 0.07 to 0.25)

$(DEPP)_2O : SiO_2$ = from 0.05 to 0.50 (preferably from 0.10 to 0.20)

$H_2O : Na_2O$ = from 50 to 600 (preferably from 150 to 400).

According to a preferred embodiment, the cationic dilution ratios, namely the molar ratios:

$r1 = H_2O : (DEPP)_2O$ (organic cationic dilution ratio)

$r2 = H_2O\ [(DEPP)_2O+Na_2O]$ (global cationic dilution ratio) are respectively equal to or higher than 180 (preferably than 225) and 100 (preferably 110).

Crystallization can be carried out under autogenous pressure, in an autoclave equipped with an anchor stirrer, in one or more steps, between 130 and 200°C for one or more days, and preferably between 160 and 200°C; after discharging the autoclave, the solid is separated from the liquid for example by filtration or centrifugation. The sources of Si, Al, Ga, Ge and Na may be compounds capable of providing the corresponding oxides, such as for instance aluminium silicates, sodium aluminates, sodium silicates, silica hydrosol, silica gel, silicic acid and sodium hydroxide; $(DEPP)^+$ ion can be provided by the hydroxide, a halide (in particular bromide or iodide), a sulphate, a phosphate or the nitrate of said cation. The zeolite can be subjected to the exchange of the original ion with the proton (from acids), or with the ammonium ion; in particular it is possible to obtain zeolites in the acid form by exchange of sodium with the ammonium ion (using for example ammonium chloride, nitrate or hydroxide) and by successive calcination of zeolite. The ammonium ion decomposes and a proton is left in the structure; the sodium ions (or the protons) of zeolite can be successively exchanged with all the cations of the Periodic System and in particular with the cations of the 8th group. The ionic exchange

can be effected with various salts, in the presence of various solvents, according to known technologies. For the reactions mentioned hereinafter, zeolites can be used in the acid form or partially exchanged with different ions; furtherly it is possible to add active components by means of other known techniques, such as dry or wet impregnation and mechanical mixing. For its use, the zeolite can be prepared in a great variety of forms, for example in granules or extrudates; in such cases it is advisable to admix the powder obtained from the synthesis with a binding material, such as clays, silica, alumina or other metal oxides. In any case, before being used, zeolites must be at least partially dehydrated, for example by a heat-treatment at high temperatures, under vacuum or not; an activation in the air at temperatures ranging from 450°C to the temperature at which the structural collapse of the zeolite starts (about 850°C) is recommendable.

As already mentioned, the invention relates also to the utilization of the zeolites obtained by the new method in processes for the conversion of hydrocarbons by reactions usually catalyzed by acids, such as cracking, hydrocracking, isomerization, aromatization, polymerization, reforming, alkylation, disproportionation and dealkylation, for which reactions a very high activity and selectivity have been determined.

Should the zeolites, obtained, according to the invention, be used for the reactions hereinabove, one could advantageously add metals having hydrogenating properties, like for instance nickel and the other metals of the VIII group; such metals can be added either by exchange or by impregnation or by any other known technology and reduce considerably the fouling of the catalyst and cause its life to be longer. Very interesting results have been obtained in the isomerization of alkylbenzenes, and in particular in the conversion of meta-xylene to ortho and para isomers. Isomerization of meta-xylene, optionally in admixture with ethylbenzene and with lesser amounts of the desired products (ortho- and para-xylene) can be advantageously conducted in the vapour phase, in the presence of hydrogen, at temperatures ranging from 250° to 450°C, preferably from 275° to 420°C, at pressures ranging from 1000 to 2000 Kilopascal and at space velocities from 1 to 10 volumes of liquid hydrocarbon per apparent volume of catalyst and per hour (LHSV = from 1 to 10, preferably from 2 to 8 $h^{-1}$).

The examples illustrate the present invention, without being however a limitation thereof.

Features common to all the examples

The X-rays analysis has been carried out by the powder diffraction method, using a wide-angle diffractometer equipped with a proportional counter with a discriminator of the pulse-height: the X-ray diffraction patterns have been obtained by radiation K $\alpha$ of copper ($\lambda = 1.54178$ Å), filtered with a nickel foil, and employing the following operative conditions:

| | |
|---|---|
| — scanning rate: | 1° $min^{-1}$, 1/4° $min^{-1}$ |
| — divergence slit: | $\gamma = 1°$ |
| — receiving slit: | $\nu = 0.1$ mm |
| — time constant: | 4 seconds. |

Heights I and the angular positions of the diffraction peaks have been deduced from the diffractograms; relative intensities 100 $I/I_0$ and interplanar distances "d" expressed in Ångstrom have been determined on the basis of height $I_0$ of the most intense diffraction peak and of diffraction angle $2\nu$ of the recorded lines. In order to determine exactly the interplanar distances, diffraction angle $2\nu$, indicated by the goniometer, has been calibrated by means of reflections (111), (311) and (620) of a high purity silicon standard ($a_0 = 5.43088$ Å). The exchange of $Na^+$ ion with other cations and the activation treatment at 540°C have provided crystalline products, the pattern of which does not show substantial variations, with respect to the $Na^+$ form, with the exception of a few little shifts in the interplanar distances and of the relative intensities of the bands. The adsorption properties of the zeolite towards vapours help to point out their structures, as suggested by Landolt G.R. [Anal. Chem. *43* (4), 613 (1971)]. In the various examples of adsorption, water, n-hexane and cyclohexane contact zeolite samples (degasified at 350°C under vacuum) at 25°C, under a pressure lower than their respective vapour pressure at 25°C (22 torr = 2993 Pa, for water, 45 torr = 6099 Pa for n-hexane and 28 torr = 3733 Pa for cyclohexane). The difference of weight before and after adsorption indicates the value of the adsorption.*

Example 1

75 g of diethyl-piperidinium bromide, 3 g of sodium aluminate and 7.5 g of NaOH were dissolved in 300 $cm^3$ of water. The solution was heated at 60—70°C, whereupon 225 g of silica sol type Ludox

*R. Le Van. Mao, React. Kinet Catal. Lett., Vol. *12* (1) 69—76 (1979)

A S® were added, and then again at a temperature from 60 to 70°C under intense stirring for further 10 minutes. The solution had the following composition by mols:

$SiO_2 = 1.144$ (DEPP)Br = 0.371

$Al_2O_3 = 0.017$ $H_2O = 25.4$

$Na_2O = 0.113$

The gel was transferred into an autoclave made of Hastelloy C® and was heated at 150°C for 8 days and then at 175°C for further 5 days, under stirring (30 rpm). After cooling, the suspension was discharged from the autoclave and filtered; the solid was washed until the washing liquid had a pH value lower than 9 and then dried at 120°C for 12 hours. The product was crystalline and exhibited the X-ray pattern of Table 1; its chemical composition was as follows (% by weight):

Si = 40.7 Na = 0.9 C = 5.7

Al = 1.2 H = 1.5 N = 0.7

After activation in the air at 540°C for 10 hours, the solid exibited the following values of vapour adsorption at 25°C (% by weight):

water = 10.4

n-hexane = 8.7 cyclohexane = 5.4.

Example 2 (form H)

The compound, washed and dried at 120°C (but not activated) as in example 1, was introduced in a flask equipped with a reflux cooler, in contact with an aqueous solution of $NH_4Cl$ at 5% by weight. The ratio between $cm^3$ of solution and grams of zeolite was equal to 19. Under moderate stirring, the flask was heated at a temperature from 70° to 80°C and kept at such temperature for 1 hour; after decantation, the same operation was repeated four times using a fresh solution of $NH_4Cl$. At the conclusion of the exchange the compound was washed with distilled water until disappearance of the $Cl^-$ ions in the washing liquid, and dried at 120°C for 12 hours. Its chemical composition (% by weight) was as follows:

Si = 4.17 Na = 0.2 C = 5.2

Al = 1.0 H = 0.9 N = 0.8

By activation of the compound in the air at 540°C for 10 hours, the so-called acid form or "H-zeolite" was obtained; the X-ray diffraction pattern is reported on Table 2.

Example 3

2.0 g of sodium aluminate, 50 g of diethylpiperidinium bromide and 5 g of NaOH were dissolved in 200 $cm^3$ of water. It was heated at 60°C and 150 g of silica sol type Ludox AS® were added; after intense stirring for 10 minutes, the gel, having the following composition by moles:

$SiO_2 = 0.763$ (DEPP)Br = 0.248

$Al_2O_3 = 0.011$ $H_2O = 16.9$

$Na_2O = 0.075$

was transferred into the autoclave of example 1, where it was heated at 150°C for 8 days and then at 190°C for further 6 days. After discharge, the solid was washed and dried as in example 1. The product exhibited the diffraction pattern recorded in Table 3; its chemical composition (% by weight) was as follows:

Si = 41.1 Na = 0.5 C = 5.4

Al = 1.15 H = 0.6 N = 0.7

The solid activated in the air at 540°C for 10 hours exhibited the following vapour adsorption at 25°C (% by weight):

water = 9.6

n-hexane = 8.2 cyclohexane = 4.5

Example 4 (form H)

The product, washed and dried at 120°C (but not activated) as in example 3, was exchanged with $NH_4^+$ (see example 2) and dried at 120°C for 12 hours. It exhibited the following chemical composition (% by weight):

Si = 41.7 Na = 0.2 C = 5.0

Al = 1.15 H = 0.8 N = 0.8

By activation of the compound in the air at 540°C for 10 hours, the so-called acid form or "H-zeolite" was obtained; the product in the acid form, showed the X-ray diffraction pattern reported on Table 4.

Example 5

50 g of diethyl-piperidinium bromide, 2 g of sodium aluminate and 5 g of NaOH were dissolved in 250 $cm^3$ of water; the solution was heated at 60—70°C, whereupon 150 g of silica sol type Ludox AS® were added. Heating of the mixture at 60°—70°C was carried on under intense stirring for further 10 minutes the solution had the following composition by moles:

$SiO_2$ = 0.763 (DEPP)Br = 0.248

$Al_2O_3$ = 0.011 $H_2O$ = 19.7

$Na_2O$ = 0.075

The gel was introduced into the autoclave of example 1 and heated at 150°C for 9 days and then at 180°C for other 5 days under stirring (30 rpm). After cooling, the suspension was discharged and filtered; the solid was washed and dried as in example 1. The product revealed the diffraction pattern shown in Table 5. Its chemical composition was as follows (% by weight):

Si = 40.1 Na = 0.8 C = 4.4

Al = 0.9 H = 0.4 N = 0.6

The solid activated in the air at 540°C for 10 hours exhibited the following data of vapour adsorption at 25°C (% by weight):

water = 7.8

n-hexane = 5.7 cyclohexane = 4.3

Example 6 (form H)

The product washed and dried at 120°C (but not activated) as in example 5, was exchanged with $NH_4^+$ (see example 2) and dried at 120°C for 12 hours.

It exhibited the following composition (% by weight):

Si = 42.5 Na = 0.2 C = 3.9

Al = 1.0 H = 0.5 N = 0.7

By activation of the product in the air at 540°C for 10 hours, the so-called acid form or "H-zeolite" was obtained; the product in acid form, showed the X-ray diffraction pattern of Table 6.

Example 7

The zeolites in the acid form obtained in examples 2, 4 and 6 were mixed with 10% by weight of bentonite, homogeneously kneaded with a suitable amount of water and extruded in pieces having a diameter of 1 mm; the product was then dried for 12 hours between 100° and 120°C and activated for 2 hours at 540°C. The resulting catalyst was employed in the isomerization of meta-xylene, either as such or in admixture with about 10% by weight of ethylbenzene (see Table 7). For this purpose, 30 $cm^3$ of catalyst were charged into a steel reactor having an inside diameter of 16 mm, electrically heated; test conditions and results obtained are recorded in Table 7. It is possible to deduce, besides high

approaches to the thermodynamic equilibrium, also the fact that the reaction product essentially consists of $C_8$ aromatics, benzene, toluene and higher polyalkyl-aromatics (having more than 8 carbon atoms); the non-aromatic liquid products, generally derived from the hydrogenation of the aromatic nucleus, were present in negligible amounts, while gaseous products (for example methane) were not revealed by the gas chromatographic analysis. Approach to the thermodynamic equilibrium means the ratio (% are by weight):

$$\frac{\text{\% of p-xylene flowing out} - \text{\% of p-xylene flowing in}}{\text{\% of p-xylene at equilibrium} - \text{\% of p-xylene flowing in}} \times 100$$

wherein the percentage of para-xylene at the equilibrium is calculated in the 3-components system; as one can see from the results, no ethylbenzene was obtained from the xylenes.

## Example 8

90 g of diethyl-piperidinium bromide, 4.5 g of sodium aluminate and 15.8 g of NaOH were dissolved in 675 $cm^3$ of water. The solution was heated at 60—70°C, whereupon 330 g of silica sol type Ludox HS® were added, and then heated again at a temperature from 60 to 70°C under intense stirring for further 10 minutes. Ludox HS® is a colloidal aqueous solution containing 30% by weight of $SiO_2$ and 0.32% $Na_2O$ as stabilizer; it differs from Ludox AS® employed in examples 1, 3 and 5, because in this latter $Na_2O$ is replaced by a 0.25% by weight of $NH_3$. The resulting mixture had the following composition by mols:

| | |
|---|---|
| $SiO_2 = 1.65$ | (DEPP)Br = 0.405 |
| $Al_2O_3 = 0.025$ | $H_2O = 50.3$ |
| $Na_2O = 0.242$ | |

The gel was transferred into the autoclave of example 1 and heated at 190°C for 4 days in only one step under stirring (30 rpm). After cooling, the suspension was discharged and filtered; the solid was then washed and dried as in example 1. The product was crystalline; its chemical composition was as follows (% by weight):

| | | |
|---|---|---|
| Si = 42.6 | Na = 0.4 | C = 3.4 |
| Al = 0.9 | H = 0.4 | N = 0.4 |

The compound, washed and dried at 120°C, was introduced into a flask equipped with a reflux cooler, in contact with an aqueous solution of $NH_4$ Cl at 5% by weight. The ratio between $cm^3$ of solution and grams of zeolite was equal to 19. Under moderate stirring, the flask was heated at a temperature from 70° to 80°C and kept at such a temperature for 1 hour; after decantation, the same operation was repeated four times using a fresh solution of $NH_4Cl$. At the conclusion of the exchange the compound was washed and dried as in example 2. Its chemical composition (% by weight) was as follows:

| | | |
|---|---|---|
| Si = 42.5 | Na = 0.2 | C = 3.3 |
| Al = 1.1 | H = 0.4 | N = 0.7 |

By activation of the compound in the air at 540°C for 10 hours, the acid form or "H-zeolite" was obtained. 35.8 parts by weight of such zeolite in acid form were admixed with 3.6 parts by weight of bentonite and homogeneously kneaded with a solution obtained by dissolving 2.15 parts by weight of nickel nitrate (hexahydrate) in 25 parts by weight of weight of water; the whole was then extruded into granules having a diameter of 1 mm. The product was dried for 12 hours between 110 and 120°C and activated for 2 hours at 540°C. The resulting catalyst was employed in the isomerization of meta-xylene. To this purpose, 30 $cm^3$ of catalyst were charged into the reactor of example 7; test conditions and results are recorded in Table 7. The X-ray diffraction pattern, before the exchange with ammonium chloride, is reported in Table 8. Zeolites showing such a X-ray diffraction pattern or the patterns reported in the Tables from 1 to 6, which are practically in overlapping with the pattern of Table 8, have been indicated by us as "MB 28" zeolites and represent a new class of zeolites. It is worthwhile to note that the two following molar ratios (cationic dilution ratios), used during the preparation:

r1 = $H_2O$: $(DEPP)_2O$, namely the organic cationic ratio
r2 = $H_2O$: $[(DEPP)_2O+Na_2O]$, namely the global ratio

are rather high, with respect to the corresponding ratios of examples 1, 3 and 5, as it can be easily deduced from the following data

| Example | r1 | r2 |
|---|---|---|
| 1 | 150.3 | 90.4 |
| 3 | 150.2 | 90.1 |
| 5 | 175.1 | 105.1 |
| 8 | 248.9 | 113.5 |
| 10 | 249.8 | 149.9 |

In particular, for the present example 8, the sodium factor (SF = r2:r1) is equal to 113.5:248.9 = 0.456.

### Example 9

The "H form" of the zeolite prepared according to example 8 was tested in an isomerization apparatus as in example 7 and at the beginning the results were better, but after a few hundreds of hours lower results were obtained, with respect to the nickel impregnated zeolite tested in example 8; it is possible therefore to state that nickel exerts a beneficial action as to the life of the catalyst.

### Example 10

50 g of diethyl-piperidinium bromide and 2 g of sodium aluminate were dissolved in 400 cm³ of water. The solution was heated at 60—70°C, whereupon 15 g of an aqueous solution of sodium silicate and 150 g of silica sol type Ludox HS® were added; the silicate solution contained 20% by weight $SiO_2$ and 23% by weight $Na_2O$ what practically corresponds to the formula of metasilicate. We heated again at a temperature from 60 to 70°C under intense stirring for further 10 minutes. The solution had the following composition by mols:

$SiO_2 = 0.83$ (DEPP)Br = 0.225

$Al_2O_3 = 0.011$ $H_2O = 28.1$

$Na_2O = 0.075$

The gel was transferred into the autoclave of example 1 and heated at 170°C for 7 days, under stirring (30 rpm). After cooling, the suspension was discharged and filtered; the solid was washed and dried as in example 1. Its chemical composition was as follows (% by weight):

Si = 40.0 Na = 0.5 C = 6.9

Al = 1.0 H = 1.1 N = 0.9

The compound, washed and dried at 120°C was introduced into a flask equipped with a reflux cooler, in contact with an aqueous solution of $NH_4Cl$ at 5% by weight. The ratio between cm³ of solution and grams of zeolite was equal to 1.9. Under moderate stirring, the flask was heated at a temperature from 70° to 80°C and kept at such temperature for 1 hour; after decantation, the same operation was repeated four times using a fresh solution of $NH_4Cl$. At the conclusion of the exchange the compound was washed and dried as in Example 1. Its chemical composition (% by weight) was as follows:

Si = 41.2 Na = 0.2 C = 6.5

Al = 1.0 H = 0.8 N = 0.9

By activation of the compound in the air at 540°C for 10 hours, the acid form or "H-zeolite" was obtained. 30 g of such "H form" of the zeolite were admixed with 3 g of bentonite and homogeneously kneaded with 25 cm³ of water; the whole was then extruded into granules having a diameter of 1 mm. The granules were then dried for 12 hours between 110 and 120°C and activated again for 2 hours at 540°C. 30 cm³ of catalyst were loaded into the reactor of example 7; test conditions and results are reported on Table 7.

Example 11

The catalyst of the preceeding example 10 was regenerated in air at 540°C for 16 hours and 15.5 g of the regenerated granules were impregnated with 18 $cm^3$ of an aqueous solution containing 0.85 g of $Ni(NO_3)_2.6H_2O$. After drying at 120°C for 12 hours the granules were loaded in the reactor of example 7 and identical operative conditions were followed as in preceeding example 10. The following results are reached:

| Results | After 24 hours | After 360 hours |
|---|---|---|
| Approach to the equilibrium (% b.w.) | 99.3 | 99.5 |
| Aromatic $C_8$ loss (% b.w.) | 2.9 | 1.1 |
| Meta-xylene | 52.7% b.w. | 53.9% b.w. |
| Para-xylene | 23.0% b.w. | 23.4% b.w. |
| Ortho-xylene | 21.5% b.w. | 21.5% b.w. |
| Ethyl-benzene | 0 | 0 |
| Benzene | traces | 0 |
| Toluene | 1.5% traces | 0.7% b.w. |
| Higher aromatics | 1.3% traces | 0.5% b.w. |

It is easy to note that the best performance, when nickel is present, is reached after many hours, what is an index of stability and of long life.

TABLE 1

| d (Å) | I/I$_o$ | d (Å) | I/I$_o$ | d (Å) | I/I$_o$ | d (Å) | I/I$_o$ | d (Å) | I/I$_o$ |
|---|---|---|---|---|---|---|---|---|---|
| 13,51 | 13 | 4,26 | 18 | 2,788 | 1 | 2,007 | 11 | 1,491 | 1 |
| 11,16 | 56 | 4,13 | 4 | 2,733 | 3 | 1,999 | 3 | 1,454 | 2 |
| 10,02 | 29 | 4,08 | 1 | 2,703 | 2 | 1,982 | 1 | 1,436 | 3 |
| 9,79 | 3 | 3,97 | 52 | 2,683 | 1 | 1,962 | 1 | 1,420 | 2 |
| 9,06 | 46 | 3,84 | 98 | 2,659 | 2 | 1,945 | 3 | 1,407 | 2 |
| 7,45 | 16 | 3,83 | 24 | 2,607 | 5 | 1,916 | 2 | 1,396 | 2 |
| 7,08 | 8 | 3,76 | 11 | 2,577 | 2 | 1,906 | 3 | 1,383 | 4 |
| 6,71 | 5 | 3,72 | 39 | 2,545 | 3 | 1,874 | 7 | 1,376 | 7 |
| 6,55 | 42 | 3,65 | 18 | 2,508 | 6 | 1,820 | 10 | 1,374 | 2 |
| 6,37 | 9 | 3,50 | 1 | 2,495 | 3 | 1,806 | 2 | 1,336 | <1 |
| 6,06 | 6 | 3,46 | 53 | 2,458 | 6 | 1,787 | 3 | 1,288 | 1 |
| 6,00 | 13 | 3,37 | 8 | 2,416 | 4 | 1,767 | 1 | 1,258 | 3 |
| 5,78 | 16 | 3,35 | 100 | 2,401 | 2 | 1,757 | 2 | 1,241 | 1 |
| 5,73 | 1 | 3,24 | 1 | 2,283 | 6 | 1,720 | 1 | 1,230 | 1 |
| 5,57 | 14 | 3,216 | 30 | 2,238 | 3 | 1,713 | 2 | 1,220 | <1 |
| 5,42 | 1 | 3,195 | 8 | 2,216 | 1 | 1,690 | 2 | 1,212 | 2 |
| 5,14 | 6 | 3,140 | 3 | 2,200 | 1 | 1,673 | 5 | 1,200 | 3 |
| 5,00 | 8 | 3,089 | 3 | 2,173 | 1 | 1,662 | 2 | | |
| 4,85 | 2 | 3,053 | 11 | 2,131 | 4 | 1,641 | 1 | | |
| 4,69 | 2 | 2,984 | 12 | 2,114 | 1 | 1,624 | 1 | | |
| 4,60 | 9 | 2,945 | 6 | 2,108 | 1 | 1,614 | 2 | | |
| 4,51 | 38 | 2,924 | 1 | 2,080 | 2 | 1,591 | 2 | | |
| 4,45 | 1 | 2,880 | 13 | 2,065 | 1 | 1,564 | 2 | | |
| 4,37 | 7 | 2,856 | 2 | 2,036 | 1 | 1,542 | 8 | | |
| — | — | — | — | 2,028 | 5 | 1,519 | 3 | | |

TABLE 2

| d (Å) | I/I$_o$ | d (Å) | I/I$_o$ | d (Å) | I/I$_o$ | d (Å) | I/I$_o$ | d (Å) | I/I$_o$ |
|---|---|---|---|---|---|---|---|---|---|
| 13,51 | 13 | 4,13 | 1 | 2,726 | 1 | 1,942 | 2 | 1,383 | 3 |
| 11,16 | 68 | 4,08 | 1 | 2,703 | 1 | 1,917 | 1 | 1,376 | 5 |
| 10,02 | 35 | 3,96 | 20 | 2,683 | 1 | 1,904 | 1 | 1,374 | 1 |
| 9,76 | 3 | 2,84 | 48 | 2,659 | 1 | 1,869 | 3 | 1,344 | 1 |
| 9,06 | 30 | 3,83 | 28 | 2,607 | 3 | 1,820 | 10 | 1,289 | 1 |
| 8,14 | 1 | 3,75 | 4 | 2,545 | 1 | 1,784 | 1 | 1,254 | 2 |
| 7,45 | 1 | 3,72 | 23 | 2,504 | 4 | 1,767 | 1 | 1,242 | 1 |
| 7,11 | 1 | 3,65 | 7 | 2,491 | 2 | 1,754 | 1 | 1,229 | 1 |
| 6,69 | 5 | 3,51 | 1 | 2,458 | 6 | 1,714 | 1 | 1,226 | 1 |
| 6,55 | 19 | 3,46 | 22 | 2,416 | 2 | 1,687 | 1 | 1,210 | 2 |
| 6,35 | 7 | 3,35 | 100 | 2,392 | 2 | 1,673 | 4 | 1,200 | 3 |
| 6,00 | 14 | 3,27 | 1 | 2,279 | 6 | 1,658 | 3 | | |
| 5,78 | 6 | 3,25 | 1 | 2,238 | 2 | 1,610 | 1 | | |
| 5,71 | 2 | 3,215 | 12 | 2,211 | 1 | 1,588 | 1 | | |
| 5,57 | 8 | 3,195 | 4 | 2,200 | 1 | 1,562 | 1 | | |
| 5,42 | 1 | 3,137 | 1 | 2,159 | 1 | 1,542 | 7 | | |
| 5,36 | 1 | 3,085 | 1 | 2,131 | 5 | 1,515 | 1 | | |
| 5,14 | 2 | 3,048 | 5 | 2,108 | 1 | 1,487 | 1 | | |
| 5,00 | 7 | 2,978 | 8 | 2,078 | <1 | 1,471 | 1 | | |
| 4,84 | 1 | 2,945 | 3 | 2,067 | <1 | 1,450 | 2 | | |
| 4,69 | 1 | 2,920 | 2 | 2,028 | 2 | 1,447 | 2 | | |
| 4,60 | 5 | 2,880 | 4 | 2,007 | 5 | 1,433 | 1 | | |
| 4,51 | 13 | 2,867 | 1 | 1,993 | 5 | 1,420 | 2 | | |
| 4,36 | 5 | 2,784 | 1 | 1,981 | <1 | 1,396 | 1 | | |
| 4,26 | 22 | 2,776 | 1 | — | — | — | — | | |

TABLE 3

| d (Å) | $I/I_o$ | d (Å) | $I/I_o$ | d (Å) | $I/I_o$ | d (Å) | $I/I_o$ | d (Å) | $I/I_o$ |
|---|---|---|---|---|---|---|---|---|---|
| 13,51 | 4 | 4,13 | 2 | 2,703 | 1 | 1,962 | 1 | 1,420 | 1 |
| 11,16 | 38 | 4,08 | 1 | 2,683 | 1 | 1,945 | 2 | 1,407 | 1 |
| 10,02 | 18 | 3,97 | 17 | 2,659 | 1 | 1,916 | 2 | 1,396 | 1 |
| 9,79 | 5 | 3,83 | 72 | 2,607 | 4 | 1,910 | 1 | 1,383 | 2 |
| 9,06 | 18 | 3,82 | 24 | 2,574 | 1 | 1,874 | 4 | 1,376 | 2 |
| 7,45 | 11 | 3,75 | 7 | 2,545 | 2 | 1,820 | 10 | 1,374 | 5 |
| 7,08 | 6 | 3,72 | 26 | 2,508 | 4 | 1,806 | 1 | 1,336 | <1 |
| 6,71 | 3 | 3,65 | 11 | 2,495 | 3 | 1,787 | 1 | 1,288 | 1 |
| 6,55 | 16 | 3,50 | 1 | 2,458 | 6 | 1,767 | 1 | 1,257 | 2 |
| 6,38 | 6 | 3,46 | 20 | 2,416 | 2 | 1,757 | 1 | 1,241 | <1 |
| 6,06 | 3 | 3,37 | 9 | 2,401 | 1 | 1,720 | 1 | 1,232 | 1 |
| 6,00 | 10 | 3,35 | 100 | 2,283 | 6 | 1,713 | 1 | 1,220 | <1 |
| 5,78 | 7 | 3,24 | 1 | 2,238 | 3 | 1,690 | 1 | 1,212 | 1 |
| 5,73 | 1 | 3,216 | 8 | 2,216 | 1 | 1,673 | 4 | 1,200 | 2 |
| 5,57 | 10 | 3,195 | 5 | 2,200 | 1 | 1,662 | 3 | | |
| 5,42 | 1 | 3,142 | 1 | 2,173 | 1 | 1,641 | <1 | | |
| 5,37 | 1 | 3,089 | 1 | 2,131 | 5 | 1,624 | <1 | | |
| 5,14 | 4 | 3,053 | 6 | 2,108 | 1 | 1,614 | 1 | | |
| 5,01 | 5 | 2,984 | 9 | 2,080 | 1 | 1,591 | 1 | | |
| 4,85 | 1 | 2,945 | 3 | 2,070 | 1 | 1,564 | 1 | | |
| 4,61 | 6 | 2,924 | 1 | 2,036 | 1 | 1,542 | 7 | | |
| 4,51 | 14 | 2,880 | 5 | 2,028 | 1 | 1,520 | 1 | | |
| 4,45 | 1 | 2,856 | 2 | 2,007 | 2 | 1,489 | 1 | | |
| 4,37 | 5 | 2,788 | 1 | 1,999 | 7 | 1,454 | 3 | | |
| 4,26 | 24 | 2,733 | 1 | 1,982 | 1 | 1,436 | 1 | | |

TABLE 4

| d (Å) | I/I$_o$ | d (Å) | I/I$_o$ | d (Å) | I/I$_o$ | d (Å) | I/I$_o$ | d (Å) | I/I$_o$ |
|---|---|---|---|---|---|---|---|---|---|
| 13,51 | 7 | 3,96 | 15 | 2,455 | 7 | 1,714 | 1 | 1,228 | 1 |
| 11,16 | 50 | 3,84 | 48 | 2,416 | 2 | 1,689 | 1 | 1,225 | 1 |
| 10,02 | 29 | 3,83 | 21 | 2,392 | 2 | 1,672 | 5 | 1,210 | 1 |
| 9,76 | 3 | 3,75 | 5 | 2,281 | 6 | 1,659 | 3 | 1,200 | 2 |
| 9,06 | 19 | 3,71 | 24 | 2,259 | 1 | 1,625 | 1 | | |
| 7,40 | 1 | 3,64 | 8 | 2,238 | 2 | 1,610 | 1 | | |
| 7,05 | 1 | 3,46 | 19 | 2,211 | 1 | 1,588 | 1 | | |
| 6,69 | 4 | 3,35 | 100 | 2,200 | <1 | 1,583 | 1 | | |
| 6,55 | 14 | 3,26 | 1 | 2,170 | 1 | 1,564 | 1 | | |
| 6,35 | 7 | 3,215 | 8 | 2,126 | 5 | 1,541 | 7 | | |
| 6,00 | 12 | 3,195 | 4 | 2,099 | 1 | 1,519 | 1 | | |
| 5,78 | 4 | 3,131 | 2 | 2,075 | 1 | 1,514 | 1 | | |
| 5,72 | 2 | 3,085 | 1 | 2,067 | 1 | 1,488 | 1 | | |
| 5,56 | 8 | 3,048 | 6 | 2,028 | 2 | 1,458 | 1 | | |
| 5,36 | 1 | 2,975 | 7 | 2,007 | 3 | 1,451 | 1 | | |
| 5,14 | 2 | 2,936 | 3 | 1,992 | 5 | 1,445 | 1 | | |
| 5,00 | 5 | 2,880 | 4 | 1,981 | 1 | 1,435 | 1 | | |
| 4,84 | 1 | 2,784 | 1 | 1,942 | 2 | 1,420 | 1 | | |
| 4,72 | <1 | 2,726 | 1 | 1,917 | 1 | 1,396 | 1 | | |
| 4,60 | 4 | 2,679 | 1 | 1,904 | 1 | 1,383 | 4 | | |
| 4,51 | 10 | 2,655 | 1 | 1,869 | 3 | 1,372 | 6 | | |
| 4,35 | 5 | 2,607 | 3 | 1,820 | 10 | 1,288 | 1 | | |
| 4,26 | 24 | 2,545 | 1 | 1,784 | 1 | 1,286 | 1 | | |
| 4,12 | 1 | 2,504 | 1 | 1,768 | 1 | 1,256 | 2 | | |
| 4,08 | 1 | 2,488 | 3 | 1,755 | 1 | 1,240 | 1 | | |

TABLE 5

| d (Å) | $I/I_o$ | d (Å) | $I/I_o$ | d (Å) | $I/I_o$ | d (Å) | $I/I_o$ | d (Å) | $I/I_o$ |
|---|---|---|---|---|---|---|---|---|---|
| 13,51 | 4 | 4,37 | 2 | 2,880 | 4 | 2,028 | <1 | 1,521 | 1 |
| 11,16 | 11 | 4,26 | 20 | 2,856 | 1 | 2,007 | 3 | 1,492 | 1 |
| 10,02 | 6 | 4,13 | 1 | 2,788 | <1 | 1,999 | 2 | 1,454 | 2 |
| 9,79 | 1 | 4,08 | <1 | 2,733 | <1 | 1,982 | 3 | 1,437 | <1 |
| 9,06 | 10 | 3,97 | 10 | 2,703 | <1 | 1,962 | <1 | 1,420 | 1 |
| 7,45 | 3 | 3,84 | 29 | 2,687 | 1 | 1,945 | 1 | 1,407 | 1 |
| 7,28 | 1 | 3,83 | 6 | 2,659 | <1 | 1,916 | 1 | 1,395 | 1 |
| 7,08 | 2 | 3,75 | 1 | 2,607 | 1 | 1,910 | 1 | 1,383 | 4 |
| 6,71 | 1 | 3,72 | 9 | 2,577 | <1 | 1,874 | 1 | 1,376 | 2 |
| 6,55 | 8 | 3,65 | 3 | 2,545 | 1 | 1,820 | 10 | 1,375 | 6 |
| 6,38 | 2 | 3,56 | <1 | 2,508 | 2 | 1,802 | 3 | 1,336 | 2 |
| 6,06 | 1 | 3,50 | <1 | 2,495 | 1 | 1,787 | 1 | 1,290 | 2 |
| 6,00 | 3 | 3,46 | 18 | 2,458 | 7 | 1,767 | 1 | 1,258 | 2 |
| 5,78 | 4 | 3,37 | 4 | 2,416 | 1 | 1,757 | 1 | 1,241 | <1 |
| 5,73 | 1 | 3,35 | 100 | 2,401 | <1 | 1,726 | 2 | 1,230 | 1 |
| 5,57 | 4 | 3,27 | <1 | 2,283 | 7 | 1,713 | <1 | 1,220 | 1 |
| 5,42 | <1 | 3,24 | <1 | 2,238 | 3 | 1,690 | <1 | 1,212 | <1 |
| 5,37 | 1 | 3,216 | 6 | 2,216 | 1 | 1,673 | 4 | 1,201 | 2 |
| 5,14 | 2 | 3,195 | 2 | 2,173 | <1 | 1,662 | 2 | | |
| 5,00 | 2 | 3,140 | 1 | 2,131 | 4 | 1,641 | <1 | | |
| 4,85 | 1 | 3,089 | <1 | 2,114 | 8 | 1,624 | <1 | | |
| 4,69 | 1 | 3,053 | 2 | 2,108 | <1 | 1,614 | 1 | | |
| 4,60 | 2 | 2,984 | 14 | 2,080 | <1 | 1,591 | 1 | | |
| 4,51 | 8 | 2,945 | 1 | 2,070 | <1 | 1,563 | 1 | | |
| 4,45 | <1 | 2,921 | <1 | 2,036 | 1 | 1,543 | 7 | | |

TABLE 6

| d (Å) | $I/I_o$ | d (Å) | $I/I_o$ | d (Å) | $I/I_o$ | d (Å) | $I/I_o$ | d (Å) | $I/I_o$ |
|---|---|---|---|---|---|---|---|---|---|
| 13,51 | 3 | 4,10 | <1 | 2,729 | 1 | 1,994 | 2 | 1,462 | 1 |
| 11,16 | 19 | 4,06 | <1 | 2,700 | 1 | 1,982 | 3 | 1,454 | 2 |
| 10,02 | 11 | 3,97 | 8 | 2,659 | <1 | 1,963 | <1 | 1,436 | <1 |
| 9,77 | 2 | 3,85 | 18 | 2,607 | 1 | 1,945 | 1 | 1,420 | 1 |
| 9,05 | 9 | 3,83 | 9 | 2,577 | <1 | 1,922 | <1 | 1,406 | <1 |
| 7,45 | 1 | 3,75 | 1 | 2,545 | 1 | 1,910 | 1 | 1,396 | 1 |
| 7,32 | <1 | 3,72 | 9 | 2,503 | 2 | 1,871 | 2 | 1,383 | 3 |
| 7,08 | <1 | 3,65 | 3 | 2,491 | 1 | 1,840 | <1 | 1,376 | 2 |
| 6,70 | 1 | 3,56 | <1 | 2,458 | 6 | 1,820 | 11 | 1,375 | 6 |
| 6,55 | 7 | 3,50 | <1 | 2,416 | 1 | 1,787 | 1 | 1,290 | 1 |
| 6,35 | 2 | 3,46 | 11 | 2,401 | 1 | 1,767 | <1 | 1,257 | 2 |
| 5,99 | 5 | 3,39 | 2 | 2,342 | <1 | 1,758 | <1 | 1,243 | <1 |
| 5,78 | 3 | 3,55 | 100 | 2,327 | <1 | 1,748 | <1 | 1,229 | 1 |
| 5,73 | 1 | 3,27 | <1 | 2,283 | 6 | 1,727 | <1 | 1,211 | 1 |
| 5,57 | 3 | 3,24 | 1 | 2,238 | 3 | 1,714 | <1 | 1,201 | 2 |
| 5,37 | <1 | 3,216 | 6 | 2,216 | <1 | 1,690 | <1 | | |
| 5,14 | 1 | 3,198 | 1 | 2,173 | <1 | 1,673 | 3 | | |
| 5,00 | 2 | 3,141 | 1 | 2,131 | 5 | 1,662 | 2 | | |
| 4,84 | <1 | 3,089 | 1 | 2,106 | <1 | 1,641 | <1 | | |
| 4,69 | <1 | 3,051 | 2 | 2,080 | <1 | 1,623 | <1 | | |
| 4,59 | 1 | 2,981 | 3 | 2,065 | <1 | 1,613 | 1 | | |
| 4,51 | 6 | 2,945 | 1 | 2,036 | <1 | 1,591 | <1 | | |
| 4,36 | 2 | 2,917 | <1 | 2,028 | 1 | 1,543 | 7 | | |
| 4,26 | 20 | 2,880 | 3 | 2,007 | 2 | 1,519 | 1 | | |
| 4,13 | <1 | 2,788 | <1 | — | — | — | — | | |

TABLE 7 (*)

| Catalyst | Feeding % by weight | | Operative conditions | | Approach equilib. | Loss $C_8$ arom. % b.w. | Conversion ethyl benzene % b.w. | % by weight of products (**) | | | | | | |
|---|---|---|---|---|---|---|---|---|---|---|---|---|---|---|
| | metaxi-lene | ethyl-benzene | LHSV ($h^{-1}$) | Temp. °C | | | | meta-xylene | para-xylene | ortho-xylene | ethyl-benzene | benzene | toluene | higher aromatics |
| Ex. 2 | 89,1 | 10,9 | 2.0 | 300 | 89,0 | 2,7 | 15,2 | 56,9 | 18,7 | 12,2 | 9,4 | 0,8 | 1,1 | 0,8 |
| „ 2 | 89,1 | 10,9 | 2,0 | 325 | 89,9 | 4,0 | 20,4 | 56,6 | 19,0 | 13,1 | 8,3 | 1,4 | 1,2 | 0,4 |
| „ 2 | 89,1 | 10,9 | 4,2 | 350 | 90,9 | 5,4 | 39,9 | 54,4 | 18,9 | 14,4 | 7,0 | 2,1 | 1,5 | 1,2 |
| „ 2 | 89,1 | 10,9 | 4,1 | 375 | 91,8 | 7,6 | 49,7 | 52,6 | 18,8 | 15,4 | 5,6 | 3,0 | 2,2 | 2,3 |
| „ 2 | 89,1 | 10,9 | 2,1 | 375 | 92,8 | 12,1 | 68,6 | 48,8 | 18,4 | 16,9 | 3,8 | 4,1 | 3,9 | 4,0 |
| „ 4 | 90,6 | 9,4 | 2,1 | 300 | 83,9 | 1.5 | 17,8 | 62,3 | 18,1 | 9,8 | 8,3 | 0,7 | 0,6 | 0,2 |
| „ 4 | 90,6 | 9,4 | 2,0 | 325 | 88,2 | 2,3 | 25,6 | 59,1 | 19,1 | 12,6 | 7,0 | 1,3 | 0,7 | 0,3 |
| „ 4 | 90,6 | 9,4 | 3,8 | 350 | 91,1 | 3,5 | 34,3 | 56,9 | 19,5 | 14,0 | 6,2 | 1,7 | 0,9 | 0,8 |
| „ 4 | 90,6 | 9,4 | 2,0 | 350 | 92,9 | 5,8 | 51,6 | 52,8 | 19,7 | 16,9 | 4,9 | 2,5 | 1,8 | 1,3 |
| „ 4 | 90,6 | 9,4 | 6,0 | 375 | 92,3 | 5,5 | 44,6 | 54,8 | 19,5 | 15,2 | 5,0 | 2,6 | 1,7 | 1,2 |
| „ 6 | 100 | — | 2,0 | 300 | 91,7 | 1,1 | / | 64,6 | 21,7 | 12,6 | 0 | 0 | 0,6 | 0,5 |
| „ 6 | 100 | — | 2,0 | 325 | 96,5 | 2,7 | / | 57.9 | 22,4 | 17,1 | 0 | <0,1 | 1,3 | 1,4 |
| „ 6 | 100 | — | 4,0 | 350 | 96,6 | 3,8 | / | 56,2 | 22,1 | 17,9 | 0 | <0,1 | 1,9 | 1,8 |
| „ 6 | 100 | — | 6,0 | 375 | 95,8 | 5,4 | / | 54,8 | 21,6 | 18,2 | 0 | 0,1 | 2,6 | 2,7 |
| „ 6 | 100 | — | 4,0 | 375 | 97,0 | 6,2 | / | 52,7 | 21,7 | 19,4 | 0 | 0,1 | 3,1 | 3,1 |
| „ 8(Ni) | 100 | — | 6,0 | 350 | 100,0 | 2,2 | / | 55,6 | 23,2 | 19,0 | 0 | 0,02 | 1,3 | 0,8 |
| „ 10 | 100 | — | 5,0 | 325 | 97,5 | 0,6 | / | 53,7 | 23,1 | 22,6 | 0 | 0 | 0,4 | 0,2 |

(*) All the tests were conducted at 15 atm. and with $H_2$/hydrocarbon molar ratios = 5.
(**) Non-aromatic liquid products = 0.1%; gaseous products: absent.
(Ni) Zeolite was impregnated with Nickel before use, in order to reach a longer life.

TABLE 8

| d (Å) | $I/I_o$ | d (Å) | $I/I_o$ | d (Å) | $I/I_o$ | d (Å) | $I/I_o$ |
|---|---|---|---|---|---|---|---|
| 13,51 | 3 | 3,84 | 30 | 2,545 | 1 | 1,713 | <1 |
| 11,16 | 13 | 3,83 | 5 | 2,508 | 1 | 1,694 | <1 |
| 10,01 | 7 | 3,75 | 4 | 2,491 | 1 | 1,673 | 4 |
| 9,79 | 2 | 3,72 | 8 | 2,458 | 7 | 1,662 | 2 |
| 9,05 | 4 | 3,65 | 6 | 2,416 | 1 | 1,622 | <1 |
| 7,45 | 3 | 3.50 | <1 | 2,397 | 1 | 1,614 | <1 |
| 7,07 | 2 | 3,46 | 6 | 2,283 | 7 | 1,591 | <1 |
| 6,71 | 1 | 3,37 | 2 | 2,238 | 3 | 1,565 | <1 |
| 6,55 | 3 | 3,35 | 100 | 2,202 | <1 | 1,543 | 7 |
| 6,38 | 3 | 3,27 | <1 | 2,170 | <1 | 1,521 | <1 |
| 6,05 | 2 | 3,24 | 1 | 2,131 | 5 | 1,491 | <1 |
| 5,99 | 3 | 3,216 | 3 | 2,114 | 1 | 1,454 | 2 |
| 5,78 | 1 | 3,192 | 3 | 2,094 | <1 | 1,420 | 1 |
| 5,71 | 2 | 3,140 | <1 | 2,075 | 1 | 1,408 | <1 |
| 5,57 | 4 | 3,099 | <1 | 2,070 | <1 | 1,395 | <1 |
| 5,42 | <1 | 3,053 | 2 | 2,040 | 1 | 1,383 | 5 |
| 5,37 | 1 | 2,980 | 4 | 2,007 | 2 | 1,376 | 2 |
| 5,14 | 1 | 2,947 | 1 | 1,995 | 3 | 1,375 | 6 |
| 5,00 | 2 | 2,921 | <1 | 1,982 | 4 | 1,335 | <1 |
| 4,85 | 1 | 2,880 | 1 | 1,947 | 1 | 1,290 | 1 |
| 4,60 | 2 | 2,856 | <1 | 1,917 | <1 | 1,258 | 2 |
| 4,51 | 3 | 2,788 | <1 | 1,912 | 1 | 1,241 | <1 |
| 4,45 | <1 | 2,729 | 1 | 1,874 | 1 | 1,230 | 1 |
| 4,36 | 2 | 2,703 | <1 | 1,820 | 11 | 1,217 | <1 |
| 4,26 | 22 | 2,687 | <1 | 1,802 | <1 | 1,211 | <1 |
| 4,13 | 1 | 2,656 | <1 | 1,787 | <1 | 1,201 | 2 |
| 4,08 | <1 | 2,607 | 1 | 1,768 | <1 | | |
| 3,97 | 5 | 2,573 | 1 | 1,757 | <1 | | |

**Claims**

1. A method of preparing zeolites having the formula:

$$(1.5 \pm 0.6)M_{2/n}O.Al_2O_3.Y\ SiO_2.\ Z\ H_2O$$

wherein Y ranges from 20 to 90, preferably from 50 to 80, Z ranges from 2 to 12, preferably from 3 to 8, M is at least one cation and n is the valence of cation M, and wherein aluminium and silicon can be optionally replaced, at least partially, by gallium and germanium respectively, and showing the X-ray diffraction pattern reported in Tables 1 to 6 or 8, said method comprising the admixing of diethyl-piperidinium hydroxide $[(DEPP)^+OH^-]$ or of a salt thereof with water and with:
a) at least one compound containing sodium;
b) at least one compound containing a metal selected from the group comprising aluminium, gallium and mixtures thereof;
c) at least one compound containing an element selected from the group comprising silicon, germanium and mixtures thereof;
the respective molar ratios, expressed as ratios of oxides, being comprised in the following ranges:
$SiO_2 : Al_2O_3$ = from 20 to 120 (preferably from 50 to 85)
$Na_2O : SiO_2$ = from 0.07 to 0.50 (preferably from 0.07 to 0.25)
$(DEPP)_2O : SiO_2$ = from 0.05 to 0.50 (preferably from 0.10 to 0.20)
$H_2O : Na_2O$ = from 50 to 600 (preferably from 150 to 400), whereafter the resulting admixture is heated until the raw materials have reacted together and the resulting product crystallizes therefrom.

2. A method according to claim 1, wherein the cationic dilution ratios, namely the molar ratios:

$r1 = H_2O: (DEPP)_2O$

$r2 = H_2O: [(DEPP)_2O + Na_2O]$

are defined as follows:

r1 = equal to or higher than 180 and preferably higher than 225

r2 = equal to or higher than 100 and preferably higher than 110.

3. A method according to claim 1, wherein the sodium factor SF, namely the ratio r2:r1 is lower than 0.50.

4. A method according to claim 1, wherein the start mixture is heated, until crystallization, in at least one step, at a temperature from 130 to 200°C and preferably from 160 to 200°C, for at least 24 and preferably 48 hours.

5. A method according to claim 1, wherein first an aqueous solution is prepared, which contains the DEPP salt, sodium aluminate and NaOH, secondly the solution is heated, preferably between 60 and 70°C and thirdly a solution containing $SiO_2$ is added.

6. A method according to claim 1, characterized in that the crystallized zeolite is washed, dried and activated by means of heating in the air at high temperature, preferably ranging from 450°C to the temperature at which structural collapse starts.

7. A method according to claim 1, characterized in that the diethyl-piperidinium salt is diethyl-piperidinium-bromide, compound a) is sodium hydroxide, compound b) is sodium aluminate and compound c) is a silica sol.

8. A method according to claim 1, characterized in that said cation M is selected, from amongst the monovalent cations, the ions of the elements of the rare earths, the ions of the elements belonging to the 2nd and 8th Groups of the Periodic System of the elements and mixtures thereof.

9. A method according to claim 8, characterized in that said monovalent cations are selected from the group comprising the alkaline ions, the hydrogen ion, the ammonium ion, the diethyl-piperidinium ion (DEPP) and mixtures thereof.

10. Use of the zeolites having the formula recited in claim 1, and showing the X-ray diffraction pattern reported in Tables 1 to 6 or 8, in a process for converting hydrocarbons by means of reactions usually catalyzed by acids, such as cracking, hydrocracking, isomerization, aromatization, polymerization, alkylation, disproportionation, reforming and dealkylation.

11. The use according to claim 10, characterized in that said isomerization is an isomerization to para- and/or ortho-xylene of meta-xylene or of mixtures of meta-xylene with its isomers.

12. The use according to claim 11, characterized in that said isomerization is effected in the vapour phase, in the presence of hydrogen, at temperatures ranging from 250°C to 450°C, preferably from 275° to 400°C, at pressures ranging from 1000 to 2000 Kilopascal and at space velocities of from 1 to 10 volumes of liquid hydrocarbons per volume of catalyst and per hour.

13. The use according to claim 11, wherein the zeolites are in the acid form.

14. The use according to claim 11, wherein the zeolite catalysts contain nickel or other metals of the VIII group.

15. The use according to claim 11 wherein the zeolites are admixed with a binder, preferably selected from the group comprising silica, alumina, clays such as bentonite, and mixtures thereof.

16. The use according to claim 11, wherein the zeolites have the X-ray diffraction pattern reported on Table 8.

17. Zeolites, designated MB28, having the formula recited in claim 1 and showing the X-ray diffraction pattern reported in Tables 1 to 6 or 8.

## Revendications

1. Procédé de préparation de zéolites ayant la formule:

$$(1,5 \pm 0,6)\ M_{2/n}\ O, Al_2O_3, Y\ SiO_2,\ Z\ H_2O$$

dans laquelle:

Y est de 20 à 90, de préférence de 50 à 80;

Z est de 2 à 12, de préférence de 3 à 8;

M est au moins un cation; et,

n est la valence du cation M,

et dans laquelle l'aluminium et le silicium peuvent être éventuellement remplacés, au moins partiellement par du gallium et du germanium respectivement, et présentant le diagramme de diffraction aux rayons X consigné sur les tableux 1 à 6 ou 8, ce procédé consistant à mélanger l'hydroxyde de diéthylpipéridinium $\{(DEPP)^+OH^-\}$ ou un sel de celui-ci avec de l'eau et avec:

a.) au moins un composé du sodium;

b.) au moins un composé d'un métal choisi dans le groupe constitué par l'aluminium, le gallium et des mélanges de ceux-ci;

c.) au moins un composé d'un élément choisi dans le groupe constitué par le silicium, le germanium et des mélanges de ceux-ci,

les rapports molaires respectifs, exprimés en tant que rapports des oxydes, étant compris dans les gammes suivantes:

$SiO_2/Al_2O_3$ = de 20 à 120 (de préférence de 50 à 85),

$Na_2O/SiO_2$ = de 0,07 à 0,50 (de préférence de 0,07 à 0,25),

$(DEPP)_2O/SiO_2$ = de 0,05 à 0,50 (de préférence de 0,10 à 0,20),

$H_2O/Na_2O$ = de 50 à 600 (de préférence de 150 à 400),

le mélange résultant étant ensuite chauffé jusqu'à ce que les matières premières aient réagis ensemble et que le produit qui en résulte cristallise à partir de ceux-ci.

2. Procédé suivant la revendication 1, caractérisé en ce que les rapports de dilution cationique, à savoir les rapports molaires:

$r_1 = H_2O/(DEPP)_2O$

$r_2 = H_2O/\{(DEPP)_2O + Na_2O\}$

sont définis comme suit:

$r_1$: égal à ou supéreur à 180 et de préférence supérieur à 225,

$r_2$: égal à ou supérieur à 100 et de préference supérieur à 110.

3. Procédé suivant la revendication 1, caractérisé en ce que le facteur de sodium SF, c'est-à-dire le rapport $r_1/r_2$ est inférieur à 0,50.

4. Procédé suivant la revendication 1, caractérisé en ce que le mélange de départ est chauffé jusqu'à cristallisation en au moins une étape à une température de 130 à 200°C, et de préférence de 160 à 200°C, pendant au moins 24 et de préférence 48 heures.

5. Procédé suivant la revendication 1, caractérisé en ce que l'on prépare tout d'abord une solution aqueuse contenant le sel de DEPP, l'aluminate de sodium et NaOH, deuxièmement chauffe la solution, de préférence entre 60 et 70°C et troisièmement ajoute une solution contenant $SiO_2$.

6. Procédé suivant la revendication 1, caractérisé en ce que la zéolite cristalline est lavée, séchée et activée par chauffage dans l'air à température élevée allant de préférence de 450°C à la température à laquelle débute l'effondrement structural.

7. Procédé suivant la revendication 1, caractérisé en ce que le sel de diéthylpipéridinium est le bromure de diéthylpipéridinium, le composé a) est l'hydroxyde de sodium, le composé b) est l'aluminate de sodium et le composé c) est un sol de silice.

8. Procédé suivant la revendication 1, caractérisé en ce que le cation M est choisi parmi les cations monovalents, les ions des éléments des terres rares, les ions des éléments appartenant au deuxième et au huitième groupes de la classification périodique des éléments et des mélanges de ceux-ci.

9. Procédé suivant la revendication 8, caractérisé en ce que les cations monovalents sont choisis parmi le groupe constitué des ions alcalins, de l'ion hydrogéne, de l'ion ammonium, de l'ion pipéridinium (DEPP) et de mélanges de ceux-ci.

10. Utilisation des zéolites ayant la formule indiquée dans la revendication 1, et présentant le diagramme de diffraction aux rayons X consigné sur les tableaux 1 à 6 ou 8, dans un procédé de conversion des hydrocarbures au moyen de réactions habituellement catalysées par des acides, telles que craquage, hydrocraquage, isomérisation, aromatisation, polymérisation, alkylation, disproportionation, réformage et désalkylation.

11. Utilisation suivant la revendication 10, caractérisé en ce que ladite isomérisation est une isomérisation en para- et/ou ortho-xylène du méta-xylène ou de mélanges de méta-xylène avec ces isomères.

12. Utilisation suivant la revendication 11, caractérisé en ce que ladite isomérisation est effectuée en phase vapeur en présence d'hydrogène à des températures allant de 250 à 450°C, de préférence de 275 à 400°C, à des pressions allant de 1.000 à 2.000 Kpascal et à des vitesses spatiales de 1 à 10 volumes d'hydrocarbures liquides par volume de catalyseur et par heure.

13. Utilisation suivant la revendication 11, caractérisé en ce que les zéolites sont sous leur forme "acide".

14. Utilisation suivant la revendication 11, caractérisé en ce que les catalyseurs de zéolites contiennent du nickel ou d'autres métaux du groupe VIII.

15. Utilisation suivant la revendication 11, caractérisé en ce que les zéolites sont mélangées avec un liant de préférence choisi dans le groupe constitué par la silice, alumine, argiles telles que bentonite et des mélanges de celles-ci.

16. Utilisation suivant la revendication 11, caractérisé en ce que les zéolites ont le diagramme de diffraction aux rayons X consigné sur le tableau 8.

17. Zéolites, appelées MB 28, ayant la formule indiquée dans la revendication 1, et présentant le diagramme de diffraction aux rayons X indiqué dans les tableaux 1 à 6 ou 8.

## Patentansprüche

1. Verfahren zur Herstellung von Zeolithen der Formel:

$$(1{,}5 \pm 0{,}6)M_{2/n}O.Al_2O_3.Y\ SiO_2.\ Z\ H_2O$$

worin Y von 20 bis 90, vorzugsweise von 50 bis 80 reicht, Z von 2 bis 12, vorzugsweise von 3 bis 8 reicht, M mindestens einen Kation und n die Wertigkeit des Kations M ist und worin Aluminium und Silicium gegebenenfalls zumindest teilweise durch Gallium bzw. Germanium ersetzt sein können, die das in den Tabellen 1 bis 6 oder 8 gezeigte Röntgenbeugungsmuster aufweisen, welches Verfahren umfaßt das Vermischen von Diethyl-piperidiniumhydroxid $[(DEPP)^+OH^-]$ oder eines Salzes davon mit Wasser und mit:

a) mindestens einer Natriumverbindung;

b) mindestens einer Verbindung eines Metalls, ausgewählt aus der Aluminium, Gallium und Gemische davon umfassenden Gruppe

c) mindestens eine Verbindung eines Elementes, ausgewählt aus der Silicium, Germanium und Gemische davon umfassenden Gruppe;

wobei die jeweiligen Molverhältnisse, ausgedrückt als Verhältnisse von Oxiden, die folgenden Bereiche umfassen:

$SiO_2$: $Al_2O_3$ = von 20 bis 120 (vorzugsweise von 50 bis 85)
$Na_2O$ : $SiO_2$ = von 0,07 bis 0,50 (vorzugsweise von 0,07 bis 0,25)
$(DEPP)_2O$: $SiO_2$ = von 0,05 bis 0,50 (vorzugsweise von 0,10 bis 0,20)
$H_2O$: $Na_2O$ = von 50 bis 600 (vorzugsweise von 150 bis 400)

worauf das erhaltene Gemisch erhitzt wird, bis die Rohmaterialien miteinander reagiert haben und das erhaltene Produkt daraus kristallisiert.

2. Verfahren nach Anspruch 1, worin die kationischen Verdünnungsverhältnisse, d.h. die Molverhältnisse:

r1 = $H_2O$: $(DEPP)_2O$
r2 = $H_2O$: $[(DEPP)_2O+Na_2O]$

wie folgt definiert sind:

r1 = gleich oder höher als 180 und vorzugsweise höher als 225
r2 = gleich oder höher als 100 und vorzugsweise höher als 110.

3. Verfahren nach Anspruch 1, worin der Natriumfaktor SF, das heißt das Verhältnis r2: r1, niedriger als 0,50 ist.

4. Verfahren nach Anspruch 1, worin das Ausgangsgemisch bis zur Kristallisation in mindestens einer Stufe mindestens 24 und vorzugsweise 48 Stunden auf eine Temperatur von 130 bis 200°C, vorzugsweise 160 bis 200°C, erhitzt wird.

5. Verfahren nach Anspruch 1, worin zuerst eine wässrige Lösung hergestellt wird, die das DEPP-Salz, Natriumaluminat und NaOH enthält, zweitens die Lösung erhitzt wird, vorzugsweise zwischen 60 und 70°C, und drittens eine $SiO_2$ enthaltende Lösung zugegeben wird.

6. Verfahren nach Anspruch 1, dadurch gekennzeichnet, daß man den kristallisierten Zeolith

wäscht, trocknet und durch Erhitzen an der Luft auf hohe Temperatur, vorzugsweise im Bereich von 450°C bis zu der Temperatur, bei der der strukturelle Zusammenbruch beginnt, aktiviert.

7. Verfahren nach Anspruch 1, dadurch gekennzeichnet, daß das Diethyl-piperidiniumsalz Diethyl-piperidiniumbromid, die Verbindung a) Natriumhydroxid, die Verbindung b) Natriumaluminat und die Verbindung c) ein Silicasol ist.

8. Verfahren nach Anspruch 1, dadurch gekennzeichnet, daß das Kation M ausgewählt ist unter einwertigen Kationen, den Ionen von Seltenerdelementen, den Ionen von Elementen der zweiten und achten Gruppe des Periodensystems und Gemischen davon.

9. Verfahren nach Anspruch 8, dadurch gekennzeichnet, daß die einwertigen Kationen ausgewählt sind aus der Gruppe, umfassend die Alkaliionen, das Wasserstoffion, das Ammoniumion, das Diethyl-piperidiniumion (DEPP) und Gemische davon.

10. Verwendung der Zeolithe der in Anspruch 1 genannten Formel, die das in den Tabellen 1 bis 6 oder 8 gezeigte Röntgenbeugungsmuster aufweisen, in einem Verfahren zur Umwandlung von Kohlenwasserstoffen mit Hilfe von Reaktionen, die gewöhnlich durch Säuren katalysiert werden, z.B. Cracken, Hydrocracken, Isomerisieren, Aromatisieren, Polymerisieren, Alkylieren, Disproportionieren, Reformieren und Dealkylieren.

11. Verwendung nach Anspruch 10, dadurch gekennzeichnet, daß die Isomerisierung eine Isomerisierung von m-Xylol oder Gemischen von m-Xylol mit seinen Isomeren zu p- und/oder o-Xylol.

12. Verwendung nach Anspruch 11, dadurch gekennzeichnet, daß die Isomerisierung in der Dampfphase in Gegenwart von Wasserstoff bei Temperaturen von 250 bis 450°C, vorzugsweise 275 bis 400°C, Drucken von 1000 bis 2000 kPa und Raumgeschwindigkeiten von 1 bis 10 Volumina flüssigen Kohlenwasserstoffen pro Volumen Katalysator und pro Stunde durchgeführt wird.

13. Verwendung nach Anspruch 11, worin die Zeolithe in der sauren Form vorliegen.

14. Verwendung nach Anspruch 11, wirin die Zeolithkatalysatoren Nickel oder andere Metalle der Gruppe VIII enthalten.

15. Verwendung nach Anspruch 11, worin die Zeolithe mit einem Bindemittel vermischt sind, das vorzugsweise ausgewählt ist aus der Gruppe, die Siliciumdioxid, Aluminiumoxid, Tone, wie Bentonit, und Gemische davon umfaßt.

16. Verwendung nach Anspruch 11, worin die Zeolithe das in Tabelle 8 gezeigte Röntgenbeugungsmuster aufweisen.

17. Zeolithe der Bezeichnung MB28 mit der in Anspruch 1 gezeigten Formel und dem in den Tabellen 1 bis 6 oder 8 gezeigten Röntgenbeugungsmuster.